# EUROPEAN PATENT APPLICATION

(11) **EP 3 050 975 A1**
(43) Date of publication of application: **03.08.2016**
(21) Application number: 14848032.0
(22) Date of filing: 26.09.2014
(51) Int. Cl.: C12Q 1/68

(54) **METHOD FOR PREDICTING THE RESPONSE TO CHEMOTHERAPY TREATMENT IN PATIENTS SUFFERING FROM COLORECTAL CANCER**

(30) Priority: 26.09.2013 ES 201331405
(71) Applicant: Servicio Andaluz de Salud, 41071 Sevilla (ES); Consejo Superior De Investigaciones Científicas (CSIC), 41013 Sevilla (ES); Fundación Pública Andaluza Para la Gestión de la Investigación en Salud en Sevilla, 41013 Sevilla (ES); Universidad de Sevilla, 41013 Sevilla (ES)
(72) Inventor: GARCÍA CARBONERO, Rocío, E-41071 Sevilla (ES); ESTÉVEZ GARCÍA, Purificación, E-41071 Sevilla (ES); PAZ-ARES RODRÍGUEZ, Luis G., E-41071 Sevilla (ES); MOLINA PINELO, Sonia, E-41013 Sevilla (ES); CARNERO MOYA, Amancio, E-41013 Sevilla (ES)
(74) Representative: Fuster, Gustavo
(86) International application number: PCT/ES2014/070731
(87) International publication number: WO 2015/044495

(57) **Abstract**

The invention relates to a method for obtaining useful data for predicting the response to chemotherapy treatment in patients suffering from colorectal cancer, which allows a specific individual quantitative identification model to be established, based on the expression profile of certain marker genes.

## Description

### FIELD OF THE INVENTION

The present invention is comprised within of the field of molecular biology and medicine. It relates specifically to a method for obtaining useful data for predicting the response to chemotherapy treatment in colorectal cancer patients, which allows establishing an individual, specific quantitative recognition pattern, which can be modified with treatment, allowing establishing groups of patients with the same diagnosis but different clinical behavior, the most suitable treatment for each patient thereby being able to be selected. Response to the treatment is predicted by means of the analysis of the *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* gene expression profile.

### PRIOR ART

Colorectal cancer (CRC) is the third most common tumor in the world with more than 1.2 million new cases diagnosed every year and is responsible for about 8% of cancer-related deaths (http://globocan.iarc.fr). About two thirds of patients are diagnosed in early stages (I-III) of the disease, potentially curable by means of surgical treatment followed by adjuvant chemotherapy or not. Nevertheless, 40% of these patients eventually relapse after surgery. Post-surgery adjuvant chemotherapy treatment reduces the risk of tumor relapse by 10-20%. Overall, it is therefore estimated that about 80% of the patients do not benefit from a chemotherapy treatment complementary to surgery, either because they are cured with surgical treatment alone or because they are unreceptive to chemotherapy treatment. Tumors not susceptible to complete surgical resection are considered incurable. In this context, chemotherapy has shown to improve in a modest but significant manner patients' quality of life and life expectancy, although at the expense of considerable financial cost and of non-negligible toxicity. Standard advanced colorectal cancer treatment consists of different combinations of fluoropyrimidines with oxaliplatin or irinotecan. These therapeutic schemes induce objective tumor responses in 40-60% of the patients with median survival of about 20 months. Over the past decade, various drugs, including drugs directed against endothelial growth factor or VEGF (bevacizumab, aflibercept) or epidermal growth factor receptor or EGFR (cetuximab, panitumumab), have been incorporated into the therapeutic arsenal with promising results. Nevertheless, despite these advancements most patients with unresectable disease eventually die due to tumor progression with a median survival of 20-24 months in the best case scenario (Vieitz et al., 2011. Clin. Transl. Oncol. 13(11), 798-804; García-Carbonero et al. 2010. Clin. Transl. Oncol., 12(11), 729-734).

The conventional prognosis factors used in conventional staging systems such as TNM, based essentially on the histopathological characteristics of the tumor and on its extent of spread in the organism, classify colorectal carcinoma patients into four large groups or stages of the disease: tumors limited to the colorectal mucosa or stage I, tumors infiltrating the entire intestinal wall or stage II, tumors invading the regional lymphatic nodes or stage III, and tumors with metastasis to distant organs or stage IV. Although these conventional classifications allow roughly differentiating subgroups of patients with different long-term prognosis, there is an enormous biological heterogeneity within said subgroups both in terms of survival and in terms of potential tumor response to chemotherapy treatment. In this context, the development of new biological markers which allow improving the ability to classify patients into prognosis/predictive subgroups is fundamental.

The elaboration of gene expression profiles using microarray technology shows a high potential in cancer research since it allows the simultaneous analysis and comparison of thousands of genes in various biological samples (for example, tumor samples) from patients affected by the same disease but with different clinical behavior (Nannini et al. 2009. Cancer Treat. Rev. 35(3), 201-209). The analysis of gene expression pattern has shown to improve diagnosis, prognosis and the predictive precision in different types of cancer (Liang et al. 2003. Nat. Rev. Cancer, 3, 869-876). For example, several studies have shown how the gene expression profiles discriminate between breast cancer with or without estrogen receptors, or between familial or sporadic breast cancer. Furthermore, previously unknown molecular subgroups have thus been described, such as the basal or luminal breast cancer cell subgroup, and have given rise to new proposals for classifying tumors of the central immune system or soft part sarcomas. However, the more appealing application from the clinical viewpoint is the possibility of differentiating between patient subgroups with different probabilities of cancer recurrence or death (Rosenwald et al., 2002. N. Engl. J. Med. 246, 1937-1947; van de Vijver He Y.D. et al., 2002. N. Engl. J. Med. 247, 1999-2009; Beer D.G. et al., 2002. Nat. Med. 8, 816-824), or with different probabilities of responding to a specific antineoplastic agent (Staunton J.E. et al., 2001. Proc. Natl. Acad. Sci 98, 10787-10792; Pusztai L. et al., 2003. The Oncologist, 8, 252-258).

It has been demonstrated in the last 2 decades that gene expression profile can differentiate between normal colonic tissue, benign adenomas and adenocarcinomas in different stages of spread of the disease, and the risk of developing CRC depending on normal colonic tissue analysis, as well as the probability of recurrence after a CRC surgical resection can also be determined (Bertucci et al., 2004. Oncogene 23, 1377-1391; Bandrés et al., 2007. Oncology Reports 17, 1089-1094). However, there has been little research on the value of this technology as a predictor of colon cancer response to chemotherapy.

Treatments for advanced CRC have limited efficacy, are costly and are not without risks for the patient. Based on the foregoing, it is fundamental to develop biomarkers which allow predicting prospectively which patients will respond to the treatment. On one hand, providing tools which allow identifying a *priori* those patients who will not benefit from a specific medical treatment would prevent the patient from suffering toxicity and from investing the money and time that it entails for receiving said treatment, while at the same time enable said patient to receive an alternative treatment with more chances of success. On the other hand, identification of biological markers predictive of tumor response can provide fundamental information concerning the molecular mechanisms determining and regulating said response and facilitate discovery of ways for manipulating the response which allow maximizing the therapeutic effect. In this context, microarray study of the gene expression pattern in patients with advanced CRC treated with chemotherapy in function of their response to same could facilitate the development of a genetic signature with unquestionable clinical usefulness.

### BRIEF DESCRIPTION OF THE INVENTION

A first aspect of the invention relates to the use of any of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes, or any of the combinations thereof for predicting the response to chemotherapy treatment in an individual suspected of suffering from colorectal cancer or diagnosed with colorectal cancer.

Another aspect of the invention relates to a method for obtaining useful data for predicting the response to chemotherapy treatment in an individual suspected of suffering from colorectal cancer or diagnosed with colorectal cancer, which comprises:
a) obtaining an isolated biological sample comprising tumor cells from the individual, and
b) detecting the amount of expression product of any of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes, or any of the combinations thereof in the isolated sample of (a).

In a preferred embodiment, the first method of the invention further comprises:
c) comparing the expression levels of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes in the sample obtained in (a) with the amount of expression detected for said genes in a reference population of individuals with a known pathological response.

Another aspect of the invention relates to a method for predicting or for the prognosis of the response to chemotherapy treatment in an individual suspected of suffering from colorectal cancer or diagnosed with colorectal cancer, comprising steps (a)-(c) of the first method of the invention, and further comprises:
d) including the individual having an expression level of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes significantly greater than the expression levels of the same genes in a reference population, in the group of individuals responding to the colorectal cancer therapy.

In a preferred embodiment of this aspect of the invention, the biological sample isolated from an individual of step (a) used for determining the expression levels of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes is obtained from the colorectal tumor tissue of the patient.

In another preferred embodiment of the invention, the determination of the expression level of genes does not need to be limited in a particular manner and can be selected from a genetic profiling method, such as a microarray, and/or a method comprising PCR (polymerase chain reaction), such as real time PCR and/or Northern blot.

More preferably, the expression product of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes is detected by means of real time quantitative PCR.

In another even more preferred embodiment of this aspect of the invention, the expression product of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes is detected by means of microarrays.

In a preferred embodiment of this aspect of the invention, the chemotherapy treatment comprises the use of platinum-based compounds, topoisomerase I-inhibiting compounds, pyrimidine analog compounds, or any of the combinations thereof. More preferably, the platinum-based compound is oxaliplatin, the topoisomerase I inhibitor is irinotecan, and the pyrimidine analog is 5-fluorouracil.

A preferred embodiment of this aspect of the invention comprises performing at least twice the sequence of steps (a) - (b) of the method of the invention on biological samples from one and the same individual isolated at different times. More preferably, the samples are obtained before and after treatment or from a chemotherapy treatment cycle.

Another aspect of the present invention relates to a kit or device, hereinafter kit or device of the invention, comprising the elements necessary for analyzing the amount of the expression product of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes.

Another aspect of the invention relates to a microarray, hereinafter microarray of the invention, comprising oligonucleotides or single-channel microarrays designed based on a known sequence or an mRNA of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes.

More preferably, the sequence of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes is the corresponding nucleotide sequence which is selected from SEQ ID NO: 1 to SEQ ID NO: XX.

Another aspect of the invention relates to the use of the kit or device of the invention or of the microarray for obtaining useful data for predicting or for the prognosis of the response to treatment in colorectal cancer patients.

### DESCRIPTION OF THE INVENTION

The present invention provides a method for obtaining useful data for predicting the response of a human subject with colorectal cancer to chemotherapy treatment, which allows establishing groups of patients affected by the same disease but with different clinical behavior. It relates to an easy and reliable *in vitro* method and tools useful for this objective, such as detection kits, devices or systems which can be used for carrying out the methods of the invention. An additional objective of the present invention lies in providing the suitable medication for an individual patient suffering from colorectal cancer based on predicting the success of the treatment.

Colorectal cancer chemotherapy treatment can consist of, but is not restricted to, the administration, in combination or alone, of medicinal products such as capecitabine, floxuridine, fluorouracil, oxaliplatin, irinotecan, etc.

Therefore, a first aspect of the invention relates to the use of any of the *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes, or any of the combinations thereof for predicting or for the prognosis of the response to chemotherapy treatment in an individual suspected of suffering from colorectal cancer or diagnosed with colorectal cancer.

Another aspect of the invention relates to the simultaneous use of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes for predicting the response to chemotherapy treatment in an individual suspected of suffering from colorectal cancer or diagnosed with colorectal cancer. However, the independent use of any of them or any of the combinations thereof may be sufficient to evaluate the response and/or tracking of said disease.

In a preferred embodiment of this aspect of the invention, the response to treatment is a response to tumor load reduction. In an alternative embodiment, the response to treatment is an improvement or absence of deterioration in the tumor stage. In another embodiment, the response to treatment is a clinical result, such as the progression-free survival or overall survival.

### METHOD FOR OBTAINING USEFUL DATA AND METHOD FOR PREDICTING THE RESPONSE TO TREATMENT OF COLORECTAL CANCER

Another aspect of the invention relates to a method for obtaining useful data, hereinafter the first method of the invention, for predicting or for the prognosis of the response to chemotherapy treatment in an individual suspected of suffering from colorectal cancer or diagnosed with colorectal cancer, comprising:
a) obtaining an isolated biological sample comprising tumor cells from the individual, and
b) detecting the amount of expression product of any of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes, or any of the combinations thereof in the isolated sample of (a).

Although the detection of the amount of expression product of any of the genes or of any of the combinations thereof can be used for predicting or for the prognosis of the response to chemotherapy treatment, in a preferred embodiment, the detection of the amount of expression product of the genes of step (b) is done simultaneously.

In another preferred embodiment, the first method of the invention further comprises:
c) comparing the expression levels of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes in the sample obtained in (a) with the amount of expression detected for said genes in a reference population of individuals with a known pathological response.

Another aspect of the invention relates to a method for predicting or for the prognosis of the response to chemotherapy treatment in an individual suspected of suffering from colorectal cancer or diagnosed with colorectal cancer, hereinafter the second method of the invention, comprising steps (a)-(c) of the first method of the invention, and further comprises:
d) including the individual having an expression level of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes significantly greater than the expression levels of the same genes in a reference population, in the group of individuals responding to the colorectal cancer chemotherapy.

Table 1 shows the expression levels obtained for said genes:

**Table 1. Expression levels in tumor cells from responsive colorectal cancer patients.**

| Gene name | Fold change in expression level | Description | Gene ID | Nucleotide sequences | Amino acid sequences |
|---|---|---|---|---|---|
| AQP1 | 2.30 | aquaporin 1 | 358 | SEQ ID NO:1 - SEQ ID NO:4 | SEQ ID NO:83 - SEQ ID NO:86 |
| CLEC3B | 3.06 | C-type lectin domain family 3, member B | 7123 | SEQ ID NO:5 | SEQ ID NO:87 |
| DCK | 1.85 | deoxycytidine kinase | 1633 | SEQ ID NO:6 - SEQ ID NO:7 | SEQ ID NO:88 - SEQ ID NO:89 |
| DEFA5 | 3.03 | defensin, alpha 5, Paneth cell-specific | 1670 | SEQ ID NO:49 | SEQ ID NO:131 |
| DNAJC3 | 2.68 | DnaJ (Hsp40) homolog, subfamily C, member 3 | 5611 | SEQ ID NO:50 | SEQ ID NO:132 |
| FBLIM1 | 1.93 | filamin binding LIM protein 1 | 54751 | SEQ ID NO:13 - SEQ ID NO:15 | SEQ ID NO:95 - SEQ ID NO:97 |
| GAS7 | 2.00 | growth arrest-specific 7 | 8522 | SEQ ID NO:16 - SEQ ID NO:19 | SEQ ID NO:98 - SEQ ID NO:101 |
| IGFBP4 | 1.74 | insulin-like growth factor binding protein 4 | 3487 | SEQ ID NO:20 | SEQ ID NO:102 |
| KSR1 | 2.00 | kinase suppressor of ras 1 | 8844 | SEQ ID NO:51 | SEQ ID NO:133 |
| LONP1 | 1.74 | Ion peptidase 1, mitochondrial | 9361 | SEQ ID NO:52 | SEQ ID NO:134 |
| MTHFD1L | 1.89 | methylenetetrahydrofolate dehydrogenase (NADP+ dependent) 1-like | 25902 | SEQ ID NO:53 - SEQ ID NO:56 | SEQ ID NO:135 - SEQ ID NO:138 |
| NAV1 | 1.84 | neuron navigator 1 | 89796 | SEQ ID NO:21 - SEQ ID NO:22 | SEQ ID NO:103 - SEQ ID NO:104 |
| NIPBL | 2.07 | Nipped-B homolog | 25836 | SEQ ID NO:57 - SEQ ID NO:58 | SEQ ID NO:139 - SEQ ID NO:140 |
| PALM2 | 2.30 | PALM2-AKAP2 readthrough | 445815 | SEQ ID NO:59 - SEQ ID NO:60 | SEQ ID NO:141 - SEQ ID NO:142 |
| PCDHGC3 | 1.99 | protocadherin gamma subfamily C, 3 | 5098 | SEQ ID NO:23 - SEQ ID NO:29 | SEQ ID NO:105 - SEQ ID NO:121 |
| PROS1 | 2.09 | protein S (alpha) | 5627 | SEQ ID NO:8 | SEQ ID NO:90 |
| RARA | 1.95 | retinoic acid receptor, alpha | 5914 | SEQ ID NO:40 - SEQ ID NO:43 | SEQ ID NO:122 - SEQ ID NO:125 |
| RSF1 | 1.74 | remodeling and spacing factor 1 | 51773 | SEQ ID NO:44 | SEQ ID NO:126 |
| TENC1 | 1.92 | tensin-like C1 domain containing phosphatase (tensin 2) | 23371 | SEQ ID NO:45 - SEQ ID NO:47 | SEQ ID NO:127 - SEQ ID NO:129 |
| TGFBR3 | 2.22 | transforming growth factor, beta receptor III | 7049 | SEQ ID NO:10 - SEQ ID NO:12 | SEQ ID NO:92 - SEQ ID NO:94 |
| TRAK2 | 2.33 | trafficking protein, kinesin binding 2 | 66008 | SEQ ID NO:48 | SEQ ID NO:130 |
| VSNL1 | 1.95 | visinin-like 1 | 7447 | SEQ ID NO:61 | SEQ ID NO:143 |
| WHSC1L1 | 2.17 | Wolf-Hirschhorn syndrome candidate 1-like 1 | 54904 | SEQ ID NO:9 | SEQ ID NO:91 |
| WWC2 | 1.91 | WW and C2 domain containing 2 | 80014 | SEQ ID NO:62 | SEQ ID NO:144 |
| FAF1 | -1.01 | Fas (TNFRSF6) associated factor | 11124 | SEQ ID NO:63 | SEQ ID NO:145 |
| FBXO9 | -1.08 | F-box protein 9 | 26268 | SEQ ID NO:64 - SEQ ID NO:66 | SEQ ID NO:146 - SEQ ID NO:148 |
| KIAA1033 | -1.11 | WASH complex subunit 7 | 23325 | SEQ ID NO:67 | SEQ ID NO:149 |
| N4BP2L1 | -1.02 | NEDD4 binding protein 2-like 1 | 90634 | SEQ ID NO:68 - SEQ ID NO:69 | SEQ ID NO:150 - SEQ ID NO:151 |
| SLC12A | -1.03 | Solute carrier family 12 (sodium/potassium/chloride transporters), member 2 | 6558 | SEQ ID NO:70 - SEQ ID NO:74 | SEQ ID NO:152 - SEQ ID NO:156 |
| STARD13 | -1.72 | StAR-related lipid transfer (START) domain containing 13 | 90627 | SEQ ID NO:75 - SEQ ID NO:80 | SEQ ID NO:157 - SEQ ID NO:162 |
| RB1 CC1 | -1.04 | RB1-inducible coiled-coil 1 | 9821 | SEQ ID NO:81 - SEQ ID NO:82 | SEQ ID NO: 162 - SEQ ID NO:164 |

In the context of the present invention, the *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes are also defined by a nucleotide or polynucleotide sequence constituting the coding sequence of the proteins listed, respectively, in the SEQ ID listed in Table 1, and would comprise many variants originating from:
a) nucleic acid molecules encoding a polypeptide comprising the amino acid sequence of the SEQ ID listed in Table 1,
b) nucleic acid molecules the complementary strand of which hybridizes with the polynucleotide sequence of a),
c) nucleic acid molecules the sequence of which differs from a) and/or b) due to genetic code degeneration,
d) nucleic acid molecules encoding a polypeptide comprising the amino acid sequence with an identity of at least 80%, 90%, 95%, 98% or 99% with the SEQ ID listed in Table 1, respectively, and in which the polypeptide encoded by said nucleic acids has the activity and structural characteristics of proteins *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1.* Among said nucleic acid molecules, the one listed in the SEQ ID indicated in Table 1 is included.

The subjects whose response is predicted are human subjects suspected of suffering from and/or have preferably been diagnosed with colorectal cancer preferably, but not limited to, advanced stages of the disease (stage IV of the TNM classification). The terms "individual", "human subject", "subject" and "patient" are therefore used interchangeably in this specification. As it is used herein, the term "individual" refers to animals, preferably mammals, and more preferably, humans. The term "individual" does not seek to be limiting in any aspect, the individual being able to be of any age, sex and having any physical condition.

Also as it is used herein, "one or more" includes one and the individualized specification of any number that is more than one, such as two, three, four, five, six, etc. As it is used herein, "more than one" or "some" includes the individualized specification of any number that is more than one, such as two, three, four, five, six, etc.

Steps (b) and/or (c) of the methods described above can be completely or partially automated, for example, by means of a piece of robotic sensing equipment for the detection of the amount of expression product of the genes in step (b) or the computerized comparison in step (c). In addition to the steps specified above, it can comprise other additional steps, for example, steps relating to sample pre-treatment or evaluation of the results obtained by means of these methods. For example, the method for predicting the response to treatment of a colorectal cancer patient can include an additional step consisting of generating a report about the results of the patient gene profile, including the probability of response to treatment of said patient diagnosed with colorectal cancer.

In a preferred embodiment of this aspect of the invention, the biological sample isolated from an individual of step (a) used for determining the expression levels of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes is obtained from the colorectal tumor tissue of the patient.

The studies conducted by the authors of the present invention have allowed obtaining information about gene expression patterns in patients affected with colorectal cancer who are responsive to treatment compared to non-responsive subjects. This has allowed obtaining a model of significant modifications defining the sensitivity to chemotherapy treatment in this type of pathology.

The amount of the expression product of the genes can be detected by any means known in the state of the art. The authors of the present invention have demonstrated that detecting the amount or the concentration of these expression products in a semi-quantitative or quantitative manner allow differentiating between the responsive individual with colorectal cancer and the non-responsive individual. The detected amount of the expression product of the genes could establish a differential profile in individuals affected by colorectal cancer, which allows subclassifying them depending on their sensitivity to chemotherapy.

The amount or concentration can be measured directly or indirectly, preferably in a semi-quantitative or quantitative manner. Direct measurement refers to measurement of the amount or concentration of the expression product of the genes based on a signal which is obtained directly from the transcripts of said genes, or from the proteins translated from said genes, and which is directly correlated with the number of RNA molecules or proteins produced by the genes. Said signal which can also be referred to as intensity signal can be obtained, for example, by measuring an intensity value of a chemical or physical property of said products. Indirect measurement includes the measurement obtained from a secondary component or a biological measurement system (for example, the measurement of cell responses, ligands, "labels" or enzyme reaction products).

As it is used herein, the term "amount" refers, but is not limited, to the absolute or relative amount of the expression products of the genes, as well as to any other value or parameter related thereto or can be derived therefrom. Said values or parameters comprise signal intensity values obtained from any of the physical or chemical properties of said expression products obtained by means of direct measurement. Additionally, said values or parameters include all those obtained by means of indirect measurement, for example, any of the measurement systems described in another part of the present document.

As it is used herein, the term "comparison" refers, but is not limited, to the comparison of the amount of the expression products of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes of the biological sample to be analyzed, also referred to as target biological sample, with an amount of the expression products of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes of one or more desirable reference samples described in another part of the present description. The reference sample can be analyzed, for example, simultaneously or consecutively, together with the target biological sample. The comparison described in section (c) of the method of the present invention can be performed manually or assisted by a computer.

The expression products of the genes will give a specific gene expression profile. "Gene expression profile" is understood as the gene profile obtained after quantifying the mRNA and/or protein produced by the genes of interest or biomarkers, i.e., by the *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes in an isolated biological sample. The expression profile of the genes is preferably established by determining the mRNA level derived from their transcription, before extraction of the total RNA present in the isolated biological sample, which can be performed by means of protocols known in the state of the art. The mRNA level derived from the transcription of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes can determined, for example, although without limitation, by means of polymerase chain reaction (PCR) amplification, reverse transcription in combination with polymerase chain reaction (RT-PCR), quantitative RT-PCR, reverse transcription in combination with ligase chain reaction (RT-LCR), or any other nucleic acid amplification method; serial analysis of gene expression (SAGE, SuperSAGE); DNA chips prepared with oligonucleotides deposited by any mechanism; DNA microarrays prepared with oligonucleotides synthesized *in situ* by means of photolithography or by any other mechanism; *in situ* hybridization using specific probes labeled with any labeling method; by means of electrophoresis gels; by means of membrane transfer and hybridization with a specific probe; by means of nuclear magnetic resonance or any other imaging diagnostic technique using paramagnetic nanoparticles or any other type of detectable nanoparticles functionalized with antibodies or by any other means. The gene expression profile could also be obtained by means of detecting and/or quantifying the proteins product of the translation of the mRNA derived from the transcription of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes, by means, for example, but without limitation, of immunodetection by Western blot. The expression of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes can be quantitatively detected, more preferably, by means of real time PCR (RT-PCR or RTqPCR). The real time detection of the amplified products can be carried out by means of using fluorescent molecules that are intercalated in the double-stranded DNA or by means of hybridization with different types of probes.

Therefore, in another preferred embodiment of the invention the detection of the expression level of the genes does not need to be limited in a particular manner and can be selected from a genetic profiling method, such as a microarray, and/or a method comprising PCR (polymerase chain reaction), such as real time PCR and/or Northern blot.

More preferably, the expression product of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes is detected by means of real time quantitative PCR and is expressed as -ΔΔCt. Real time quantitative PCR (RQ-PCR) is a sensitive and reproducible gene expression quantification technique which can be used particularly for gene profile expression in cells and tissues. Any method can be used for evaluating the results of RT-PCR and the ΔΔCt method is preferred. The ΔΔCt method is described in detail in Livak et al. (Methods 2001, 25, 402-408). (Ct = Threshold values of the cycle). When carrying out the present invention to practice, the ΔΔCt method as described by Livak et al. (Methods 2001, 25:402-408) must preferably be used. The ΔΔCt method will involve a "sample from the control" and a "sample from the subject". The "sample from the subject" is a sample originating from the subject to be analyzed. A target gene (hereinafter the gene of interest) and an endogenous control gene (as described below) are included in each sample for PCR amplification from aliquots (usually serial dilutions). Usually, several replicates of each diluted concentration are used to derive the amplification efficiency. PCR amplification efficiency can be defined as the percentage of amplification (from 0 to 1). Durante the qPCR reaction, a piece of software usually measures the number of cycles of each sample in which the fluorescence intersects an arbitrary line (indicative of PCR amplification), the threshold. This point of intersection is the Ct value. More diluted samples will intersect subsequent Ct values. To quantify the gene expression of a particular gene, the Ct of a nucleic acid originating from the gene of interest is subtracted from the Ct of the nucleic acid originating from the endogenous control in the same sample for normalizing variation in the amount and quality of RNA between different samples and obtaining the relative expression (with respect to the endogenous control) of each of the samples, the "sample from the subject" and the "sample from the control". Optionally, this is carried out in duplicate, triplicate, quadruplicate and in a similar manner, respectively. A ΔCt value of the control can be suitably obtained by calculating the average of the ΔCt values obtained from samples of a control group consisting of a few individuals with which the values of the "sample from the subject" will be compared. The control group (from which the average value is calculated) consists of individuals suitable for the respective (comparison) purposes. From this disclosure, the skilled person will learn that a suitable control group is for a specific purpose. In a particular embodiment, the present invention can be carried out to practice omitting the determination of the ΔCt value of the control group, i.e., determining (only) the ΔCt value of the "sample from the subject" and then subsequently comparing this with the respective average ΔCt value of the control indicated in the examples.

In another still more preferred embodiment of this aspect of the invention, the expression product of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes is detected by means of microarrays.

The reference amount is obtained from the values of constitutive expression of genes in a group of healthy individuals or of the expression of the genes in the group of individuals before being subjected to treatment.

The reference amount will be, for example, in the case of differentiating between patients affected by colorectal cancer responsive to treatment, the constitutive expression of the gene in a control group of non-responsive individuals.

On the other hand, the reference sample or samples can be, for example, obtained from tumor tissue of a colorectal cancer patient who did not respond to treatment.

In another preferred embodiment of this aspect of the present invention, the reference amount is obtained from a reference sample. The reference amount can also be obtained, for example, from the normal distribution limits of an amount found in samples obtained from a population of non-responsive individuals with colorectal cancer, by means of well known statistical techniques. In another preferred embodiment, the reference sample is obtained from the patients before and after treatment. Therefore, a preferred embodiment of this aspect of the invention comprises performing at least twice the sequence of steps (a) - (b) of the method of the invention on biological samples from one and the same individual isolated at different times. More preferably, the samples are obtained before and after treatment.

The inventors have demonstrated that the expression levels of one or more of these genes can be indicative of the response of a subject to treatment. In a preferred embodiment of this aspect of the invention, the response is a significant tumor load reduction evaluated using universal standard objective radiological criteria (RECIST criteria). In an alternative embodiment, the response is a clinical result, such as the tumor progression-free survival.

The evaluation of the complete response and/or partial response is an important factor for determining the stage of an individual patient. It is therefore necessary to estimate the total tumor load in the baseline value and use this as a comparative element for subsequent measurements that are usually carried out according to the RECIST criteria (version 1.1), Eisenhauer et al., 2009. Eur. J. Cancer, 45 (228-247), defined as follows:
1. Complete response (CR): Disappearance of all measureable and evaluable evidence of the disease
2. Partial response (PR): At least a 30% decrease in the sum of diameters of target lesions, taking as reference the baseline sum diameters.
3. Progressive disease (PD): At least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm. (Note: the appearance of one or more new lesions is also considered progression).
4. Stable disease (SD): Neither sufficient shrinkage to qualify for PR nor sufficient increase to qualify for PD.

Throughout the present invention, the terms CR, PR, PD and SD are defined according to preceding definitions 1 to 4 taken from the revised RECIST Guidelines of Eisenhauer et al., 2009. Eur. J. Cancer, 45 228-247.

In another preferred embodiment, the neoadjuvant treatment comprises the use of radiotherapy. In another even more preferred embodiment, said treatment comprises administering chemotherapy with fluoropyrimidine-type compounds and an additional agent.

The chemotherapy, the response to which is to be predicted, comprises standard chemotherapy regimens in colorectal cancer treatment, fluoropyrimidine-based regimens. Chemotherapy is understood as cancer treatment with an antineoplastic drug or with a combination of said drugs. Without intending to be bound to any theory in particular, it is understood that chemotherapy usually acts by eliminating cells that divide rapidly, which is one of the main properties of most cancerous cells. For treatment of patients suffering from colorectal cancer, chemotherapy preferably comprises administering fluoropyrimidines which are thought to act like antimetabolites. The examples of fluoropyrimidine compounds are capecitabine, floxuridine and fluorouracil (5-FU), which is the most preferred one in the present invention. Said fluoropyrimidine can be administered alone or in combination with an additional agent, polychemotherapy being more effective in general although also more toxic. The additional agent can be a molecule capable of interacting with DNA, such as in inhibiting DNA synthesis and/or preventing DNA from unwinding. In a preferred embodiment of the present invention, the chemotherapy can comprise administering oxaliplatin and fluoropyrimidine, or irinotecan and fluoropyrimidine. The cytotoxicity of oxaliplatin (patent document US 4,169,846) is thought to result in the inhibition of DNA synthesis in cancerous cells. *In vivo* studies have shown that oxaliplatin has anti-tumor activity against colon carcinoma due to its (undirected) cytotoxic effect. Irinotecan on the other hand is a semi-synthetic analog of natural alkaloid camptothecin, which is thought to act by preventing the unwinding of DNA through inhibition of topoisomerase 1. Oxaliplatin and irinotecan are therefore thought to exert their function intervening with the DNA.

Therefore, in an even more preferred embodiment the chemotherapy is a therapy which comprises administering platinum-based compounds, topoisomerase I-inhibiting compounds, a pyrimidine analog compound, or any of the combinations thereof. More preferably, the platinum-based compound is oxaliplatin, the topoisomerase I inhibitor is irinotecan, and the pyrimidine analog is 5-fluorouracil.

Another aspect of the invention relates to a method for tracking the progression of colorectal cancer in an individual, hereinafter third method of the invention, which comprises performing at least twice the sequence of steps (a) - (b) according to the first or second method of the invention, on biological samples from one and the same individual isolated at different times.

### KIT OF THE INVENTION

Another aspect of the present invention relates to a kit or device, hereinafter kit or device of the invention, comprising the elements necessary for analyzing the amount of the expression product of *AQP, CLEC3B, DCK, DEFA5, DNAJC3, FBLIM1,* GAS7, *IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes.

The kit is based on the predictive power of the method of the present invention. As mentioned above, the reference indicator value for the lack of response of each specific gene can be determined before carrying out the method of the present invention. In the particular case of the kit, the reference indicator value for the lack of response (and/or a reference indicator value for the response) can be provided. With the aid of the kit, the expression of each gene can be calculated, i.e., with respect to the control samples provided by way of examples above. The control can also therefore be comprised in the kit.

The kit of the invention more preferably comprises means necessary for comparing the amount detected in step (b) with a reference amount. Said kit can contain all those reagents necessary for analyzing the amount of the expression product of *AQP, CLEC3B, DCK, DEFA5, DNAJC3, FBLIM1,* GAS7, *IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes by means of any of the methods described above in this document. The kit can further include, without any type of limitation, buffers, agents to prevent contamination, protein degradation inhibitors, etc. On the other hand, the kit can include all the supports and containers necessary for putting it into practice and optimization. Preferably, the kit further comprises the instructions for carrying out any of the methods of the invention.

More preferably, the kit or device of the invention comprises the primers and probes obtained from the gene sequences of the invention, such as those which are also described in Table 1. Since a single gene may be useful for predicting or for the prognosis of the response to treatment in colorectal cancer patients, the kit can contain the probe/probes and primers useful for quantifying the expression of said gene, or any of the combinations of *AQP, CLEC3B, DCK, DEFA5, DNAJC3, FBLIM1,* GAS7, *IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes.

In particular embodiments, the kit is selected from (a) a kit suitable for PCR, (b) a kit suitable for Northern blot and (c) a kit suitable for microarray analysis. Any of two or more of these embodiments can be combined such that the kit can comprise, for example, both of (a) and (c).

In the case of (a) a kit suitable for PCR, this PCR is usually real time quantitative PCR (RQ-PCR), a sensitive and reproducible gene expression quantification technique. In this case, the kit preferably additionally comprises primers and probes and oligonucleotides/oligonucleotides of the kit. These reagents can be optionally comprised in the kit.

Northern blot involves the use of electrophoresis for separating RNA samples by size and the subsequent detection with oligonucleotides/oligonucleotides (hybridization probe) complementary with (part of) the target sequence of the RNA of interest.

It is also possible that the oligonucleotide/oligonucleotides are immobilized on spots on a (preferably solid) surface. In one of its embodiments, the kit comprises a microarray. An RNA microarray is an array on a solid substrate (typically a glass slide or a cell in a thin silicon film) that evaluates different large amounts of RNA that are detectable by specific probes immobilized on spots on a substrate solid. Each spot contains a specific nucleic acid sequence, usually a DNA sequence as probes (or indicators). Although the number of spots is not limited in any way, there is a preferred embodiment in which the microarray is customized to the methods of the invention. In one embodiment, said customized array comprises fifty spots or less, such as thirty spots or less, including twenty spots or less.

### MICROARRAY OF THE INVENTION

Therefore, another aspect of the invention relates to a microarray, hereinafter microarray of the invention, comprising oligonucleotides or single-channel microarrays designed based on a known sequence or an mRNA of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes. More preferably, the sequence of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes is the nucleotide sequence indicated for each of them in Table 1, respectively.

The use of the kit is not particularly limited, although the use thereof is preferred in any of the methods of the invention or in any of the embodiments thereof. Therefore, another aspect of the invention relates to the use of the kit or device of the invention for obtaining useful data for the prognosis of or for predicting the response to neoadjuvant chemotherapy in individuals with colorectal cancer.

Another aspect of the invention relates to a computer readable storage medium comprising program instructions capable of making a computer carry out the steps of any of the methods of the invention (any of the first, second or third method of the invention).

Another aspect of the invention relates to a transmissible signal comprising program instructions capable of making a computer carry out the steps of any of the methods of the invention.

The terms "polynucleotide" and "nucleic acid" are used herein interchangeably, referring to polymeric forms of nucleotides of any length, both ribonucleotides (RNA) and deoxyribonucleotides (DNA). The terms "amino acid sequence", "peptide", "oligopeptide", "polypeptide" and "protein" are used herein interchangeably and refer to a polymeric form of amino acids of any length, which can be coding or non-coding, chemically or biochemically modified.

Throughout the description and the claims the word "comprises" and variants thereof do not intend to exclude other technical features, supplements, components or steps. For persons skilled in the art, other objects, advantages and features of the invention will be inferred in part from the description and in part from the practice of the invention. The following examples and drawings are provided by way of illustration and they are not meant to limit the present invention.

### BRIEF DESCRIPTION OF THE DRAWINGS

In order to help better understand the features of the invention according to a preferred practical embodiment thereof and to complement this description, a set of illustrative and non-limiting drawings is attached as an integral part thereof. In these drawings:

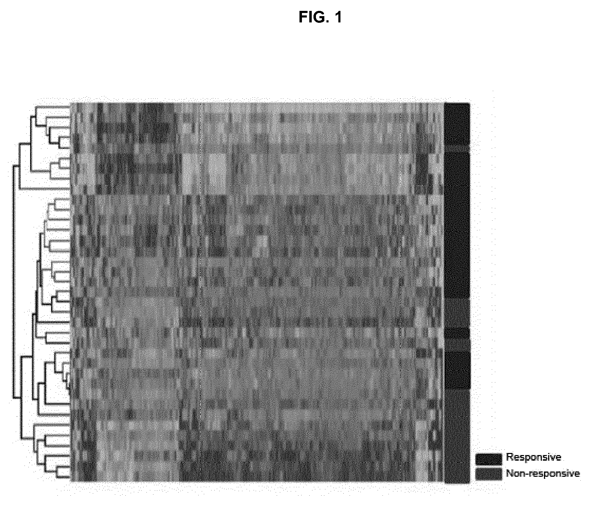

Figure 1 shows the supervised hierarchical cluster analysis showing the differentially expressed genes in patients who achieved an objective response to chemotherapy (Yes: CR and PR; blue) with respect to patients who do not respond to chemotherapy (No: SD or PD; red). The genes in red indicate overexpression and the genes in green indicate underexpression.

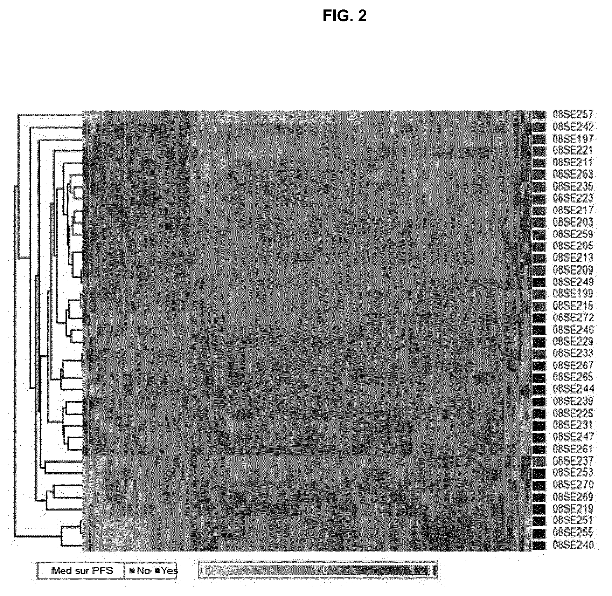

Figure 2 shows the supervised hierarchical cluster analysis showing the differentially expressed genes in patients who achieved a progression-free survival (PFS) greater than the mean time of PFS (Yes; blue) compared to patients with PFS less than the mean (No; red). The genes in red indicate overexpression and the genes in green indicate underexpression.

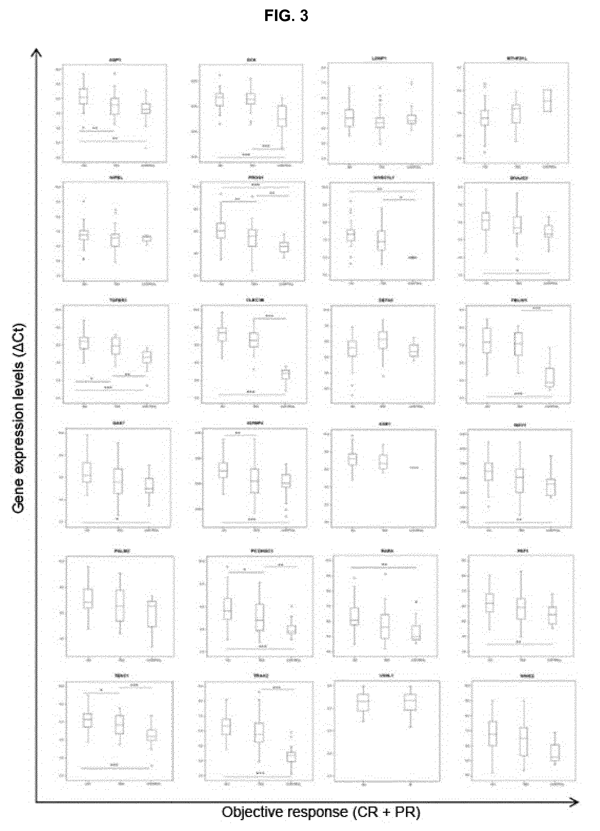

Figure 3 shows mean ΔCt values of genes validated in patients with an objective response to chemotherapy (Yes) compared to non-responsive patients (No). *p-value<0.05. **p-value<0.01. ***p-value<0.001. The data derived from RT-qPCR are presented as ΔCt values with higher values for lower RNA expression.

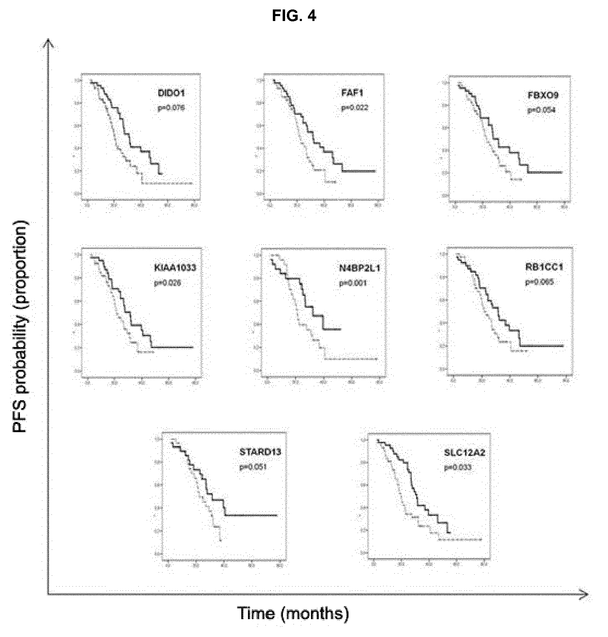

Figure 4 shows the clinical result considering the progression-free survival (PFS) of patients using gene expression levels. The solid black line represents patients with a higher gene expression level (with respect to the mean). The discontinuous black line represents patients with lower gene expression levels.

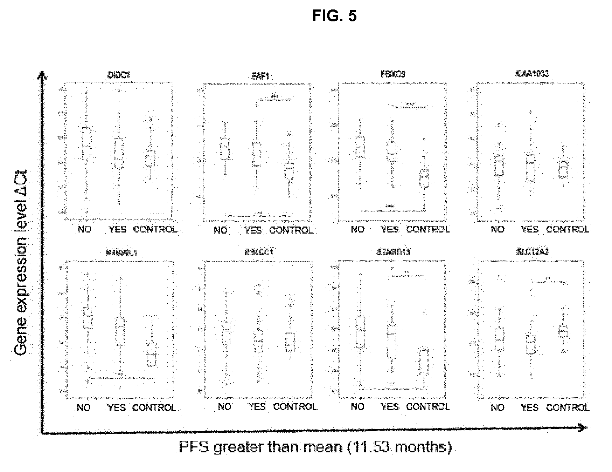

Figure 5 shows mean ΔCt values of genes validated in patients with progression-free survival (PFS) greater than the mean (11.53 months) [Yes] compared with patients with lower PFS [No]. *p-value<0.05; **p-value<0.01; ***p-value<0.001. The data derived from RT-qPCR are presented as ΔCt values with higher values for lower RNA expression.

### EXAMPLES OF THE INVENTION

The invention will be illustrated below by means of assays conducted by the inventors clearly showing the specificity and effectiveness of the methods of the invention for obtaining useful data in the prediction of the response to treatment of said disease.

### Materials and methods

The study protocol has been approved by the ethics committee of the participating centers and with a written consent form from all the patients. A total of 159 tumors from patients diagnosed with advanced CRC were used as training (n=40) and validation (n=119). Samples complying with the following criteria were included in the study: (1) confirmed primary diagnosis of CRC; (2) the patients were treated with at least one chemotherapy line for the advanced disease and the response was evaluated; (3) suitable frozen tissues available for molecular assays (a proportion of tumor cells >50% is required). The training set consisted of 40 CRC samples from Hospital Marqués de Valdecilla, Santander, Spain. The validation set consisted of 119 samples from 4 different hospitals (Hospital Virgen del Rocío of Seville (Spain), Hospital Virgen de la Victoria of Malaga (Spain), Hospital Virgen de la Merced of Osuna (Spain) and Hospital Marqués de Valdecilla of Santander (Spain), and 86 tumor samples (40 from the training set and 46 from recently treated CRC patients) were included and 33 normal tissue samples from CRC patients were used as controls. The distribution of the demographic and clinical-pathological characteristics of the initial and validation cohorts is shown in Table 2, and the histological characteristics of the tumor samples are outlined in Table 3. There were no significant differences between both groups.

**Table 2. Demographic data, clinical characteristics and treatment in the training and validation sets**

| | Training set (n=40) | Validation set (n=86) | |
|---|---|---|---|
| Characteristics | N (%) | N (%) | P |
| Age (years) | | | 0.417 |
| • Median (range) | 61 (47-79) | 65 (42-80) | |
| Sex | | | 1.00 |
| • Female | 16 (40) | 33 (38.4) | |
| • Male | 24 (60) | 53 (61.6) | |
| Metastasis in the diagnosis | | | 0.625 |
| • Yes | 34 (85) | 69 (80.3) | |
| • No | 6(15) | 17 (19.7) | |

| Metastasis/advanced disease | | | |
|---|---|---|---|
| Functional state | | | 0.306 |
| • 0-1 | 40 (100) | 82 (95.3) | |
| • 2 | 0(0) | 4 (4.7) | |
| Laboratory parameters (Median (range)) | | | |
| • CEA (ng/mL) | 62.5 (1-4007) | 28 (1-1448) | |
| • Hemoglobin (g/dL) | 12 (2-15) | 12(2-15) | |
| • Alkaline phosphatase (IU/L) | 107 (48-620) | 96 (43-16189) | |
| • Lactate dehydrogenase (IU/L) | 441 (273-2692) | 355 (109-2692) | |

| Initial chemotherapy (CT) reqimens | | | |
|---|---|---|---|
| Oxaliplatin-based chemotherapy | | | |
| • Oxaliplatin+FP | 18 (45) | 39 (45.4) | |
| • Oxaliplatin+FP+AntiEGFR | 8 (20) | 14 (16.3) | |
| • Oxaliplatin+FP+AntiEGFR/AntiVEGFR | 9 (22.5) | 14(16.3) | |
| Irinotecan-based chemotherapy | | | |
| • Irinotecan+FP | 0(0) | 9 (10.5) | |
| • Irinotecan+FP+AntiEGFR | 0(0) | 1 (1.2) | |
| • Irinotecan+FP+AntiEGFR+AntiEGFR/AntiVEGFR | 0(0) | 1 (1.2) | |
| Triple (Oxaliplatin+ Irinotecan+FP) | 5 (12.5) | 4 (4.6) | |
| Only FP | 0(0) | 3 (3.4) | |

| Response | | | |
|---|---|---|---|
| Tumor response to initial CT | | | 0.441 |
| • Yes (CR+PR) | 25 (62.5) | 46 (53.5) | |
| • No (SD+PD) | 15 (37.5) | 40 (46.5) | |
| Tumor progression after initial CT | | | 0.068 |
| • Yes | 27 (67.5) | 71 (82.6) | |
| • No | 13 (32.5) | 15 (17.4) | |
| Surgical treatment of relapse or metastasis with complete resection | | | 0.378 |
| • Yes | 12 (30) | 19 (22.1) | |
| • No | 28 (70) | 67 (77.9) | |

| Result | | | |
|---|---|---|---|
| Tracking of living patients (months) | | | 0.586 |
| • Median (range) | 31.9 (12.9-77-57) | 30.8 (5.50-77.57) | |
| Patient's condition during last contact | | | |
| • Dead | 17 (42.5) | 49 (57) | |
| • Living with tumor | 22 (55) | 31 (36) | |
| • Living without tumor | 1 (2.5) | 6(7) | |
| *CEA: carcinoembryonic antigen; EGFR: epidermal growth factor receptor; VEGF: vascular endothelial growth factor; VEGFR: vascular endothelial growth factor receptor; FP: fluoropyrimidines; CR: complete response; PR: partial response; SD: stable disease; PD: progressive disease.* | | | |

**Table 3. Characteristics of the tumors in the training and validation sets**

| | Training set (n=40) | Validation set (n=86) | |
|---|---|---|---|
| Characteristics | N (%) | N (%) | P |
| Sample type | | | |
| • Surgical part | 38 (95) | 85 (98.8) | |
| • Endoscopic biopsy | 1 (2.5) | 1 (1.2) | |
| • Others | 1 (2.5) | 0(0) | |

| Tumor localization | | | |
|---|---|---|---|
| Location of the primary tumor | | | 0.834 |
| • Colon | 28 (70) | 62 (72.1) | |
| • Rectum | 12 (30) | 24 (27.9) | |
| Location of metastasis | | | |
| • Liver | 33 (82.5) | 57 (66.3) | 0.062 |
| • Lung | 12 (30) | 22 (25.6) | 0.668 |
| • Peritoneal carcinomatosis | 6(15) | 16 (18.6) | 0.802 |
| • Others | 1 (2.5) | 24 (27.9) | |

| Histological characteristics | | | |
|---|---|---|---|
| Histology | | | 0.306 |
| • Adenocarcinoma | 40 (100) | 82 (95.3) | |
| • Mucin carcinoma | | 4 (4.7) | |
| Tumor differentiation | | | |
| • Well differentiated | 14 (35) | 38 (44.2) | |
| • Moderately differentiated | 8 (20) | 28 (32.6) | |
| • Poorly differentiated | 15 (37.5) | 15 (17.4) | |
| • Unknown | 3 (7.5) | 5 (5.8) | |
| Lymphovascular invasion | | | |
| • Yes | 23 (57.5) | 30 (34.9) | |
| • No | 3 (7.5) | 15 (17.4) | |
| • Unknown | 14 (35) | 41 (47.7) | |
| Condition of K-ras | | | 0.338 |
| • Wild-type | 25 (62.5) | 45 (52.3) | |
| • Mutated | 15 (37.5) | 41 (47.7) | |

### RNA samples

The tumor tissue samples were ground to fine powder under liquid nitrogen using a pestle and mortar. Homogenization was achieved using QIAshredder homogenizers and the total RNA was extracted using the RNeasy mini kit (both kits from Qiagen Inc; Valencia, CA, USA). The amount and quality of the RNA were estimated by means of agarose gel and spectrophotometric measurements. When it was not possible due to the small amount of RNA, they were estimated using Bioanalyzer 2100 (Eukaryote total RNA Nano or Pico kit: Agilent Technologies; Santa Clara, CA, USA). From the 40 initial samples, RNA suitable in quality and amount could be obtained in 37 cases (93%).

### Gene expression profile microarray

The microarray experiments were developed using the Human Whole Genome U133 Plus 2.0 array (Affymetrix; Santa Clara, CA, USA) containing 54675 probes of human genes. The double-stranded DNA was synthesized using One-Cycle cDNA Synthesis kit (Affymetrix), according to the manufacturer's recommendations. The total RNA (2 µg) was first reverse transcribed using a T-7 Oligo (dT) primer promoter for obtaining a single-stranded cDNA and then double-stranded cDNA which was purified with GeneChip Cleanup Module (Affymetrix) and used as a template in the subsequent *in vitro* transcription reaction. The labeled cRNA was also purified with GeneChip Cleanup Module and spectrophotometrically quantified. The purified cRNA was fragmented in fragmentation buffer (5x buffer: 200 mM Tris-acetate (pH 8.1) + 500 mM KOAc + 150 mM MgOAc) and hybridized to the microarray in 200 µl of hybridization solution containing 15 µg of labeled target in 1x Mes buffer (0.1 M Mes + 1.0 M NaCl + 20 mM ETA + 0.01% Tween 20) and 0.1 mg/ml of herring sperm DNA, 10% DMSO, 0.5 mg/ml BSA, 50 pM of B2 control oligonucleotide and 1x eukaryotic hybridization controls (nioB, bioC, bioD, cre). Both the B2 control oligonucleotide and the eukaryotic hybridization controls were obtained from Affymetrix. The hybridization mixture was applied to Human Genome U133 Plus 2.0 array (Affymetrix), which includes the entire human genome with 54613 sets of probes. The arrays were placed in the 640 hybridization oven (Affymetrix) at 45°C for 16 hours, rotating at 60 rpm. After hybridization, the arrays were washed with 6x SSPE-T (0.9 M NaCl + 60 mM NaH₂PO₄ + 6 mM AEDT + 0.01 % Tween 20) at 30°C over a fluid surface (FS400 Affymetrix) in 10 cycles of 2 mixes per cycle, and then with 0.1 M Mes + 0.1 M NaCl + 0.01% Tween 20 at 50°C in 6 cycles of 15 mixes per cycle. The arrays were then stained for 5 minutes at 35°C with a streptavidin-phycoerythrin conjugate (molecular probes), followed by 10 washing cycles of 4 mixes per cycle with 6x SSPE-T. To increase the signals, the arrays were complementarily stained with a solution of anti-streptavidin antibodies for 5 minutes, followed by 5 minutes of staining with a streptavidin-phycoerythrin conjugate. After 15 washing cycles of 4 mixes per cycle, the arrays were scanned using GC300 laser confocal scanner (Affymetrix).

### Validation of differentially expressed genes in CRC

The total RNA was extracted from the tumor tissue samples with mirVanaRNA isolation kit (Ambion, Austin, TX, USA), according to the manufacturer's instructions. The collected total RNA was determined using Nanodrop ND-100025 spectrophotometer (Nanodrop Tech, DE, USA). The customized 7900 HT Taqman® low density arrays (TLDA) with microfluidic cards having 161 individual assays were ordered from Applied Biosystems (Applied Biosystems; Carlsbad, CA, USA).

The TLDAs were developed in a 2-step process: briefly during the first step, 800 ng (50 ng/16 µl) of the total RNA were reverse transcribed using Megaplex RT primers and TaqMan RNA reverse transcription kit in a total volume of 20 µl. The reactions were incubated in a G-Storm Thermal Cycler (Gene Technologies, Essex, UK) for 5 minutes at 25°C, 30 minutes at 42°C, and one minute at 50°C for 40 cycles, maintained for 5 minutes at 85°C and then at 4°C. In the second step, 450 µL of the cDNA sample and 450 mL of the Taqman Universal PCR master mix were loaded into full ports, on the TLDA microfluidic card. The card was briefly centrifuged for 1 minute at (280 grams) to distribute the samples in the multiple wells connected to the full ports and then sealed to prevent contamination between wells. The TLDA cards were handled and analyzed using the ABI PRISM® 7900HT sequence detection protocol (Applied Biosystems). The reactions were incubated at 95°C for 10 minutes, followed by 40 cycles of 15 seconds at 95°C and one minute at 60°C.

### Statistical analysis

Clinical result variables. Statistics were used to characterize the most relevant clinical parameters. The association of categorical variables was examined using the chi-square test or Fisher's exact test. To evaluate the distribution of the continuous variables between the study groups, the parametric test (t-test) and non-parametric test (Kruskal-Wallis test) were used when suitable.

The tumor response was evaluated using conventional methods according to the RECIST 1.0 standard criteria: A complete response (CR) was defined as the disappearance of all measureable and evaluable evidence of the disease; a partial response (PR) was defined as a decrease equal to or greater than 30% in the sum of the longest diameters of target lesions; stable disease (SD) was considered when the tumor load decreased less than 30% or increased less than 20%; and progressive disease (PD) was indicated by an increase greater than 20% in the sum of the longest diameters of target lesions or the appearance of a new lesion. The patients were classified in 2 groups according to the best response to chemotherapy: those who achieved an objective response (responsive patients [R]: CR + PR) and those that did not (non-responsive patients [NR]: SD + PD). The progression-free survival (PFS) was defined as the time elapsed since the start of first-line chemotherapy until the first evidence of disease progression. The overall survival (OS) was calculated from the start of the therapy for advanced disease until the date of death from any cause. The Kaplan-Meier product-limit method was used to estimate the dependent time variables (PFS and OS), and the differences observed between the subgroups of patients were evaluated using the log-rank test. P<0.05 was considered significant. All the analyses were developed using the statistical package for social sciences software (SPSS 15.0 for Windows, SPSS Inc, Chicago, IL).

### Microarrays

The microarray image data was analyzed using the GeneChip Operating Software (GCOS 1.4 Affymetrix). Partek Genomics Suite v7.3.1 (Partek Inc.; St. Louis, MO, USA) was used for statistical analysis. The quality of the arrays was evaluated using parameters P called %, outlier array and non-scale normalized standard error. The data was then processed through the RMA (robust multichip average), method for obtaining individual intensity values in each array, then normalized and filtered to eliminate non-informative sequences (including control sequences, those having a hybridization signal close to background and those without changes in expression in all the samples). Finally, 9619 sequences were selected for generating the worklist. A linear regression model using the false discovery rate (abbreviated as FDR), main component analysis (MCA) and grouping techniques were used for comparing the gene expression profile between R and NR patients after first-line metastatic chemotherapy, and considering the PFS later.

### Real time quantitative polymerase chain reaction (RT-qPCR) analysis

The target gene expression was normalized in relation to the GAPDH expression. The threshold cycle (abbreviated as Ct) values were calculated using SDS software v.2.3 (Applied Biosystems) using automatic baseline conditions and a threshold of 0.2. The expression of each target gene was calculated in relation to an endogenous GAPDH control. The data is presented as gene expression target = 2ΔCt, with ΔCt = (Ct Ct-GAPDH target gene). This method is a suitable way for analyzing relative changes in gene expression of real time quantitative PCR experiments (Applied Biosystems user Bulletin no. 2 (P/N 4303859)). The gene expression was calculated using the Real-Time Statminer® software v.4.2 (Integromics, Inc). This software performs a moderated t-test between the groups (R and NR) and corrects same using the Benjamini-Hochberg algorithm (Benjamini Y, *et al.,* 1995) with FDR established in the value of 5%. For the purpose of this study, significant gene expression was considered when the gene had been detected at least in 50% of the patients in each compared group. Furthermore, in situations in which the gene is not detectable and the Ct values are beyond the maximum Ct32, the Statminer® software establishes a value equal to the maximum.

### RESULTS

The comparison of the relative gene expression levels between patients responsive (23 patients; 62%) and patients non-responsive (14 patients; 38%) to chemotherapy are depicted in Figure 1.

Expression differences (p<0.05) were seen in 595 genes (6.2%) from the 9619 final sequences selected after the analysis. By using the FDR test, 5% of said genes (480 genes) are considered as false positive findings. In a supervised cluster, deregulated gene expression and PFS as a response marker were detected by detecting differential associated expression profiles (Figure 2).

Based on both analyses, selecting those genes (n=161) with the most significant p-values and the deregulation thereof was consistent in the response to chemotherapy and in the survival analysis to be validated using qRT-PCR in an independent cohort of CRC patients and non-tumor control samples.

### Identification of the gene expression pattern in CRC patients according to the response to chemotherapy

Twenty-four of the 161 genes were confirmed as differentially expressed genes between patients who achieved an objective response to chemotherapy (R: CR + PR) with respect to those who did not (NR: SD + PD) (p<0.05) after qRT-PCR using the Statminer® software: *AQP1, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1* and *WWC2.*

**Table 4. Genes differentially expressed in response to chemotherapy in metastatic colorectal carcinoma patients according to real time PCR analysis**

| Gene | Gene ID | R vs. NR(-ΔΔCt) | Adjusted p-values* |
|---|---|---|---|
| *AQP1* | 358 | 1.200 | 0.025 |
| *CLEC3B* | 7123 | 1.614 | 0.006 |
| *DCK* | 1633 | 0.888 | 0.044 |
| *DEFA5* | 1670 | 1.599 | 0.017 |
| *DNAJC3* | 5611 | 1.421 | 0.006 |
| *FBLIM1* | 54751 | 0.947 | 0.038 |
| *GAS7* | 8522 | 1.000 | 0.034 |
| *IGFBP4* | 3487 | 0.803 | 0.025 |
| *KSR1* | 8844 | 0.997 | 0.034 |
| *LONP1* | 9361 | 0.800 | 0.034 |
| *MTHFD1L* | 25902 | 0.915 | 0.044 |
| *NAV1* | 89796 | 0.881 | 0.038 |
| *NIPBL* | 25836 | 1.049 | 0.026 |
| *PALM2* | 445815 | 1.203 | 0.024 |
| *PCDHGC3* | 5098 | 0.991 | 0.038 |
| *PROS1* | 5627 | 1.064 | 0.026 |
| *RARA* | 5914 | 0.960 | 0.034 |
| *RSF1* | 51773 | 0.796 | 0.044 |
| *TENC1* | 23371 | 0.942 | 0.038 |
| *TGFBR3* | 7049 | 1.150 | 0.023 |
| *TRAK2* | 66008 | 1.220 | 0.017 |
| *VSNL1* | 7447 | 0.966 | 0.034 |
| *WHSC1L1* | 54904 | 1.116 | 0.026 |
| *WWC2* | 80014 | 0.932 | 0.044 |

| | | | |
|---|---|---|---|
| *The patients were classified into two groups. i.e., patients responsive to chemotherapy (R), including patients with complete response or partial response, and non-responsive patients (NR), including patients with stable disease or progressive disease based on the change in lesion size.* *Gene ID: GenBank accession number* **The p-values obtained were adjusted for multiple tests using the Benjamini-Hochberg adjustment.* | | | |

To further evaluate the differences in potentially predictive gene expression of the response to chemotherapy, the gene expression levels of samples from R patients, NR patients and non-tumor controls were compared. The results are depicted in Figure 3. Significant differences (p<0.05) were found in 15/24 genes: *AQP, CLEC3B, DCK, PROS1, WHSC1L1, TGFBR3, FBLIM1, GAS7, IGFBP4, NAV1, PCDHGC3, RARA, RSF1, TENC1* and *TRAK2.* Additionally, the DNAJC3 gene showed a significance limit (p=0.076).

### Identification of gene expression patterns in colon cancer patients according to progression-free survival (PFS)

The Kaplan-Meier estimator estimates the PFS according to the gene expression levels in patients grouped as above or below the mean time (11,53 months) and showed 4 genes with differential expression profile (p<0.05); FAF1, KIAA1033, N4BP2L1 and SLC12A2. Another 4 genes showed significance limit and were also considered using more analyses: DIDO1 (p=0.076), FBXO9 (p=0.054), RB1CC1 (p=0.065) and STARD13 (p=0.051).

**Table 5. Genes differentially expressed by progression-free survival in metastatic colorectal carcinoma patients according to real time PCR analysis**

| Gene | Gene ID | PFS_LM vs. PFS_HM (-ΔΔCt) | p-values* (95% CI) |
|---|---|---|---|
| *DIDO1* | 11083 | -0.020 | 0.076 |
| *FAF1* | 11124 | -0.021 | 0.022 |
| *FBXO9* | 26268 | -0.116 | 0.054 |
| *KIAA1033* | 23325 | -0.148 | 0.026 |
| *N4BP2L1* | 90634 | -0.025 | 0.001 |
| *SLC12A2* | 6558 | -0.047 | 0.033 |
| *STARD13* | 90627 | -0.784 | 0.051 |
| *RB1CC1* | 9821 | -0.052 | 0.065 |

| | | | |
|---|---|---|---|
| *The patients were classified into two groups. In patients with survival time less than the progression of the median (PFS_LM) or greater than the median (PFS_HM)* **PFS was estimated by means of the Kaplan-Meier method and the p-values were evaluated by means of the log-rank test.* *Gene ID: GenBank accession number, CI: confidence interval.* | | | |

These gene expression levels between patients grouped as above or below the mean time of PFS and non-tumor control samples were then compared. Significant differences are shown in 5/8 genes: *N4BP2L1, STARD13, FAF1, FBXO9* and *SLC12A2.* (Figure 4)

## Claims

1. Simultaneous use of *AQP, CLEC3B, DCK, DEFA5, DNAJC3, FBLlM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes for predicting the response to chemotherapy treatment in an individual suspected of suffering from colorectal cancer or diagnosed with colorectal cancer.

2. A method for obtaining useful data for predicting the response to chemotherapy treatment in an individual suspected of suffering from colorectal cancer or diagnosed with colorectal cancer, which comprises:
a. obtaining an isolated biological sample comprising cells from the individual, and
b. detecting the amount of expression product of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes in the isolated sample of (a).

3. The method according to claim 3, which further comprises:
c. comparing the expression levels of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes in the sample obtained in (a), with the amount of expression detected for said genes in a reference population of individuals with a known pathological response.

4. A method for predicting the response to chemotherapy treatment in an individual suspected of suffering from colorectal cancer or diagnosed with colorectal cancer, which comprises steps (a)-(c) according to any of claims 2-3, and further comprises:
d. including the individual having an amount of expression of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1*, *WWC2, FAF1, FBXO9, KIAA1033, N4BP2L1, SLC12A, STARD13* and *RB1CC1* genes significantly greater than the expression levels of the same genes in a reference population, in the group of individuals responding to the colorectal cancer chemotherapy.

5. The method according to any of claims 2-4, where the sample is obtained from a colorectal tumor.

6. The method according to any of claims 2-5, wherein the amount of expression product of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes is obtained by means of:
a. a genetic profiling method, such as a microarray, and/or
b. a method comprising PCR, such as real time PCR; and/or
c. Northern blot

7. The method according to the preceding claim, where the amount of expression product of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes is obtained by means of real time quantitative PCR.

8. The method according to claim 7, where the expression product is detected by means of microarrays.

9. The method for predicting the response to chemotherapy treatment in an individual suspected of suffering from colorectal cancer or diagnosed with colorectal cancer according to any of claims 2-9, where the chemotherapy treatment comprises the use of platinum-based compounds, topoisomerase I-inhibiting compounds, pyrimidine analog compounds, or any of the combinations thereof.

10. The method for predicting the response to chemotherapy treatment in an individual suspected of suffering from colorectal cancer or diagnosed with colorectal cancer according to the preceding claim, where the platinum-based compound is oxaliplatin, the topoisomerase I inhibitor is irinotecan, and the pyrimidine analog is 5-fluorouracil.

11. A method for tracking the progression of colorectal cancer which comprises performing at least twice the sequence of steps (a) and (c) according to any of claims 3-10, on biological samples from one and the same individual isolated at different times.

12. A kit or device according to the preceding claim, comprising the primers and probes designed based on the nucleotide sequences of Table 1.

13. A microarray comprising oligonucleotides or single-channel microarrays designed based on a known sequence or an mRNA of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1*, *SLC12A, STARD13* and *RB1CC1* genes.

14. The microarray according to the preceding claim, where the sequence of *AQP, CLEC3B, DCK, DEFA5, DNAJC3, FBLIM1, GAS7, IGFBP4, KSR1, LONP1, MTHFD1L, NAV1, NIPBL, PALM2, PCDHGC3, PROS1, RARA, RSF1, TENC1, TGFBR3, TRAK2, VSNL1, WHSC1L1, WWC2, FAF1, FBXO9, KIAA1033, N4BP2L1, SLC12A, STARD13* and *RB1CC1* genes is the nucleotide sequence SEQ ID listed in Table 1, respectively.

15. Use of the kit or device according to any of claims 12-13, or of the microarray according to any of claims 14-15, for obtaining useful data for predicting the response to treatment in colorectal cancer patients.
